(19) Europäisches Patentamt
European Patent Office
Office européen des brevets

(11) Veröffentlichungsnummer : **0 412 415 B1**

(12) # EUROPÄISCHE PATENTSCHRIFT

(45) Veröffentlichungstag der Patentschrift : **29.07.92 Patentblatt 92/31**

(51) Int. Cl.$^5$ : **B01J 37/02,** B01J 23/64, B01J 23/44, C07C 29/17

(21) Anmeldenummer : **90114738.9**

(22) Anmeldetag : **01.08.90**

(54) **Palladiumkatalysatoren.**

(30) Priorität : **11.08.89 DE 3926561**

(43) Veröffentlichungstag der Anmeldung : **13.02.91 Patentblatt 91/07**

(45) Bekanntmachung des Hinweises auf die Patenterteilung : **29.07.92 Patentblatt 92/31**

(84) Benannte Vertragsstaaten : **BE CH DE ES FR GB IT LI NL**

(56) Entgegenhaltungen :
**EP-A- 0 198 435**
**FR-A- 2 351 703**
**GB-A- 1 103 442**
**US-A- 4 608 362**

(73) Patentinhaber : **BASF Aktiengesellschaft**
**Carl-Bosch-Strasse 38**
**W-6700 Ludwigshafen (DE)**

(72) Erfinder : **Broecker, Franz Josef, Dr.**
**Schwanthaler Allee 20**
**W-6700 Ludwigshafen (DE)**
Erfinder : **Arnold, Lothar, Dr.**
**Hausackerweg 11**
**W-6900 Heidelberg (DE)**
Erfinder : **Grafen, Paul, Dr.**
**Suedtiroler Ring 20**
**W-6719 Weisenheim (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die vorliegende Erfindung betrifft Palladiumkatalysatoren, die durch Bedampfen eines Trägermaterials mit metallischem Palladium, Behandlung der aufgedampften Palladiumschicht mit einem metallischen Inhibitor durch Bedampfen und thermische Behandlung bei Temperaturen von 300 bis 800°C herstellbar sind.

Aus der GB-A-871 804 sind Palladiumkatalysatoren, die mit Salzlösungen der Metalle Zn, Cd, Hg, Ga, In oder Tl behandelt wurden, bekannt. Diese Katalysatoren eignen sich für eine verbesserte selektive Hydrierung von Acetylenverbindungen. Zusätzlich zu dieser Behandlung mit Metallsalzlösungen wird die Hydrierung in Anwesenheit eines Amins durchgeführt.

Aus der US-A-3 192 168 ist ein mit einem Zinnsalz teilweise desaktivierter Palladiumkatalysator bekannt, der als Trägermaterial Calciumcarbonat enthält. Die partielle Vergiftung des Palladiumkatalysators erfolgt durch Tränken bei erhöhter Temperatur mit Zinnacetat bzw. Zinnchlorid.

Aus der DE-A-24 31 929 sind Palladium enthaltende Katalysatoren bekannt, die zusätzlich eines der Elemente Zink oder Cadmium und wenigstens eines der Elemente Wismut oder Tellur oder sowohl Zink als auch Cadmium enthalten. Als Katalysatorträger wird Aluminiumoxid oder Bimsstein verwendet. Diese eignen sich für ein Verfahren zur Herstellung von But-2-en-1,4-diol durch Hydrieren von Butindiol.

Die partielle Hydrierung von Dreifachbindungen zu olefinischen Doppelbindungen ist mit einer Übersicht der technisch verwendeteten Katalysatorsysteme aus M. Freifelder, "Practical Catalytic Hydrogenation", Wiley-Interscience, New York, 1971, S. 84 bis 126 bekannt.

Für die selektive Hydrierung der Dreifachbindung in Vitamin- und Riechstoff-Vorprodukten werden üblicherweise Lindlar-Katalysatoren, d.h. durch Blei modifizierte Palladiumkatalysatoren eingesetzt (Helv. Chim. Acta, 35 (1952), 446-450). In bestimmten Fällen werden diese noch mittels Schwefelverbindungen (Thiodiethylenglykol) desaktiviert (Neth. Appl. 6 506 92)8.

Die zuvor beschriebenen Hydrierverfahren werden in der Technik hauptsächlich in Suspensionsfahrweise ausgeübt. So wird häufig bei dieser Fahrweise nach einer bestimmten Wasserstoffaufnahme die Wasserstoffzufuhr gestoppt und damit eine weitere Hydrierung unterbunden.

Nachteilig an den bislang bekannten Verfahren waren die nicht ausreichenden Selektivitäten.

Es bestand daher die Aufgabe, neue Palladiumkatalysatoren zu entwickeln, den zuvor genannten Nachteilen abzuhelfen, und ein verbessertes Verfahren zur selektive Hydrierung von Verbindungen mit Dreifachbindung bereitzustellen.

Demgemäß wurden neue Palladiumkatalysatoren gefunden, welche dadurch erhältlich sind, daß man ein Trägermaterial mit metallischem Palladium bedampft, die aufgedampfte Palladiumschicht mit einem metallischen Inhibitor durch Bedampfen behandelt, und eine thermische Behandlung bei Temperaturen von 300 bis 800°C anschließt, sowie deren Verwendung zur gezielten Hydrierung von Dreifachbindungen zu olefinischen Doppelbindungen.

Als Inhibitoren können Zinn, Blei, Zink, Cadmium, Antimon und/oder Wismut verwendet werden. Vorzugsweise benutzt man Wismut. Das Trägermaterial wird mit Palladium und dem Inhibitor gleichzeitig oder nacheinander bedampft und der so erhaltene Katalysatorvorläufer getempert und dadurch zum eigentlichen Katalysator formiert.

Als Vakuumaufdampftechniken kommen alle bekannten Beschichtungsverfahren in Betracht, insbesondere die thermische Verdampfung. Man kann aber auch eine sogenannte Flash-Verdampfung, eine Kathodenzerstäubung sowie Sputtern benutzen. Die thermische Verdampfung kann durch direkte oder indirekte elektrische Heizung erfolgen. Vorzugsweise benutzt man die Elektronenstrahlverdampfung. Bei dieser Methode wird das zu verdampfende Metall in einem Tiegel mittels eines Elektronenstrahls oberflächlich so stark erhitzt, daß es verdampft.

Das so erhaltene Katalysatorsystem aus Träger, Aktivkomponente und Inhibitor wird anschließend bei Temperaturen von 300 bis 800, vorzugsweise 500 bis 700°C getempert und formiert. Nach dieser Formierung wird die Katalysatorfolie bzw. das Katalysatornetz bzw. das Katalysatorgewebe zweckmäßigerweise für den Einbau in den Hydrierreaktor zu einem Monolithen bzw. zu Formkörpern wie z. B. Sulzer®Packungen verformt. Dadurch lassen sich die gewünschten Strömungsverhältnisse im Reaktor herstellen.

Bevorzugt verwendet man metallische Netze, Folien oder Gewebe als Träger. Es ist aber auch möglich, Gewebe aus anorganischen Materialien, wie z. B. $Al_2O_3$ bzw. $SiO_2$ oder Kombinationen beider, zu verwenden. Außerdem kann man Gewebe aus Kohlefasern oder Kunststoffen einsetzen.

Besonders vorteilhaft für unsere Aufgabenstellung ist die Verwendung eines hitzebeständigen Edelstahlgewebes mit den Legierungsbestandteilen Fe, Cr und Al (Beschreibung in DIN 17 470). Dieses Gewebe wird zunächst durch Tempern an der Luft bei Temperaturen von 800 bis 1 000, vorzugsweise 900°C, in das eigentliche Trägermaterial umgewandelt. Getempert wird 2 bis 20 Stunden, vorzugsweise 4 bis 10 Stunden.

Der so erhaltene Träger wird in einer Vakuumbedampfungsanlage kontinuierlich bedampft. Dieses erfolgt

beispielsweise derart, daß man die Aktivkomponente Palladium im Vakuum bei $10^{-2}$ bis $10^{-10}$, vorzugsweise $10^{-4}$ bis $10^{-8}$ Torr, mittels eines Elektronenstrahls so stark erhitzt, daß das Metall aus dem Tiegel heraus verdampft und sich auf dem Träger kondensiert. Das Gewebe wird während des Aufdampfprozesses mittels einer Wickelvorrichtung gleichmäßig über die Verdampfungsquelle bewegt. Dabei wird die Anordnung so gestaltet, daß der größte Teil des Dampfstromes auf dem Trägermaterial kondensiert. In einem zweiten Bedampfungsschritt wird anschließend das Inhibitormetall aufgebracht. Zweckmäßigerweise erfolgt dies mit einer zweiten Verdampfungsquelle.

Der so hergestellte Monolithkatalysator wird in den Hydrierreaktor, der beispielsweise aus einem Doppelmantelrohr besteht, eingebaut. Für den Katalysatortest wird eine bestimmte Menge Hydro-Dehydrolinalool vorgelegt und mit einer Kreislaufpumpe über einen Vorheizer von oben in den Reaktor geleitet. Vor dem Reaktor wird Wasserstoff mit einem bestimmten Partialdruck zudosiert. Das zu hydrierende Produkt rieselt dann unter dem eingestellten $H_2$-Partialdruck über den Katalysator. Die Flüssigkeitsmenge wird so bemessen, daß eine Querschnittsbelastung von 20 bis 80, vorzugsweise 40 bis 70 [$m^3/m^2$ x h] erreicht wird. Zur Erzielung einer optimalen Hydrodynamik an der Katalysatorgrenzfläche wird der Wasserstoff vorzugsweise im Kreis gefahren. Die Durchführung der Katalysatorherstellung und die selektive Hydrierung einer Dreifachbindung werden in den folgenden Beispielen näher erläutert. Die selektive Hydrierung der Dreifachbindung wird mit Hydro-Dehydrolinalool (HDHL), welches nach folgender Reaktionsgleichung in Hydro-Linalool (HLIN) umgewandelt wird, dargestellt.

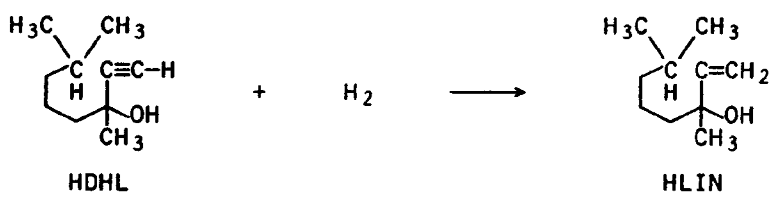

Wichtig ist bei dieser Hydrierung, daß das HDHL vollständig umgesetzt wird.

Beispiele

Beispiel 1

Katalysatorherstellung

Ein glattes Edelstahlgewebe (Werkstoffnummer 1.4767) mit einer Maschenweite von 180 μ und einem Drahtdurchmesesr von 110 μ wird zunächst im Ultraschallbad gereinigt und anschließend 7 h bei 900°C an der Luft getempert. Ein 15 cm breiter Gewebestreifen wird auf die in einer UHV-Bedampfungsanlage installierte Wickelvorrichtung aufgespannt und anschließend kontinuierlich bei einem Vakuum von $10^{-6}$ Torr mit 2 nm Pd bedampft. Durch Rückspulen des Gewebes wird dieses in einem zweiten Bedampfungsschritt mit 0,7 nm Bi belegt. Nach dem Aufdampfen wird das Katalysatorvorprodukt 30 min bei 600°C in einer Elektromuffel formiert. Dazu wird der Temperofen in 40 min auf 600°C aufgeheizt, 30 min auf dieser Temperatur gehalten und dann abgeschaltet. Nach dem Erkalten wird der Katalysator aus der Muffel entnommen und zu einem Monolithen verformt. Zur Herstellung des Monolithen werden 41,5 cm glattes Gewebe mit einer Zahnradwalze wellenartig verformt und mit 38 cm glattem Gewebe zusammengelegt und aufgerollt. Man erhält so einen Monolithkatalysator mit einem Volumen von 57 $cm^3$.

Beispiel 2

Hydrierung im Differentialreaktor

Zur selektiven Hydrierung von HDHL zu HLIN wird der Katalysator in ein Doppelmantelrohr eingebaut. Der Außenmantel des Reaktionsrohres wird mit einem Thermostaten verbunden. Unterhalb des Reaktors befindet sich ein Abscheider mit einem Gasabgang für das Kreisgas und einem Abgang zur Flüssigkeitskreislaufpumpe. Ferner können Produktproben zur Analyse entnommen werden. Aus dem Abscheider wird die zu hydrierende Flüssigkeit mit der Kreislaufpumpe über einen Vorheizer zum Reaktorkopf gepumpt. Der Vorheizer wird so reguliert, daß am Reaktoreingang eine Temperatur von 80°C erreicht wird. Hinter dem Vorheizer wird der Wasserstoff, der mit einer Menge von 67 Nl/h und einem Partialdruck von 895 Torr im Kreis gepumpt wird, zudosiert.

Die Kreislaufpumpe für das HDHL wird so eingestellt, daß eine Querschnittsbelastung von 60 [$m^3/m^2$ x h] eingehalten wird. In 7 Stunden wird das HDHL bis auf 7,34 % in HLIN überführt. Nun wird der H2-Partialdruck auf 80 Torr gesenkt, indem man den reinen Wasserstoff durch ein Gasgemisch aus Stickstoff und Wasserstoff ersetzt. Bei diesem verringerten $H_2$-Partialdruck von 80 Torr wird in 3 Stunden und 20 Minuten das HDHL zu 100 % umgesetzt. Bei diesem 100%igen Umsatz erhält man eine Selektivität bezogen auf HLIN von 99,3 %.

Beispiel 3

Kontinuierliche Hydrierung

Die kontinuierliche Hydrierung von HDHL zu HLIN wird in 2 Stufen durchgeführt. Dazu werden 2 Reaktoren benutzt, von denen der erste mit Produktrückführung und der zweite ohne betrieben wird. Der benötigte Wasserstoff wird, wie in Beispiel 2, im Kreis gefahren. Die Hydrierreaktoren bestehen aus Doppelmantelrohren, in denen der Katalysator in Form eines monolithischen Formkörpers eingebaut ist. Unterhalb des ersten Reaktors befindet sich ein Abscheider mit einer Standhaltung und Abgängen für das Kreisgas, die Produktrückführung und den Produktaustrag, der direkt zum 2. Reaktor geleitet wird. Wie in Beispiel 2, wird auch hier beim ersten Reaktor das rückgeführte Produkt mit der Kreislaufpumpe über den Vorheizer zum Reaktorkopf gepumpt. Vor dem Vorheizer wird kontinuierlich HDHL zugeführt und nach dem Vorheizer Wasserstoff in das Hydriergemisch aus HDHL und rückgeführtem Produkt mittels der Kreisgaspumpe eingeschleust. Das Hydrierprodukt der ersten Hydrierstufe wird über einen Vorheizer zum Kopf des zweiten Reaktors geleitet. Nach dem Vorheizen wird auch hier, wie beim ersten Reaktor, das Kreisgas zudosiert. Das Kreisgas besteht hier aus einem $H_2/N_2$-Gemisch mit einem bestimmten $H_2$-Partialdruck. Im 2. Reaktor wird das HDHL zu 100 % hydriert.

Für die erste Hydrierstufe gelten folgende Reaktionsbedingungen:

1. Stufe:

| | |
|---|---|
| Reaktionstemperatur | : 80°C |
| $H_2$-Partialdruck | : 900 Torr |
| Katalysatorbelastung | : 1,1 [kgHDHL/lKat. x h] |
| Querschnittsbelastung | : 60 [$m^3/m^2$ x h] |
| Kreisgasmenge | : 1,2 [$Nm^3$/lKat. x h] |

Nach dem ersten Reaktor enthält das Hydrierprodukt noch 14,8 % HDHL, die im zweiten Reaktor vollständig hydriert werden. Dazu sind die folgenden Reaktionsbedingungen notwendig:

2. Stufe:

| | |
|---|---|
| Reaktionstemperatur | : 80°C |
| $H_2$-Partialdruck | : 80 Torr |
| Katalysatorbelastung | : 0,7 [kg/lKat. x h] |
| Querschnittsbelastung | : 60 [$m^3/m^2$ x h] |
| Kreisgaszusammensetzung | : 10 % $H_2$; 90 % $N_2$ |
| Kreisgasmenge | : 1,2 [$Nm^3$/lKat. x h] |

Nach der 2. Hydrierstufe ist das HDHL zu 100 % umgesetzt. Die Selektivität in Bezug auf HLIN beträgt 99,5 %.

## Patentansprüche

1. Palladiumkatalysator, dadurch erhältlich, daß man ein Trägermaterial mit metallischem Palladium bedampft, die aufgedampfte Palladiumschicht mit einem metallischen Inhibitor durch Bedampfen behandelt und eine thermische Behandlung bei Temperaturen von 300 bis 800°C anschließt.

2. Palladiumkatalysator nach Anspruch 1, dadurch gekennzeichnet, daß das Trägermaterial ein Eisen, Chrom und/oder Aluminium enthaltender Edelstahl ist.

3. Palladiumkatalysator nach Anspruch 1, dadurch gekennzeichnet, daß das Trägermaterial netz-, gewebe- oder folienartig ist.

4. Palladiumkatalysator nach Anspruch 1, dadurch gekennzeichnet, daß das Trägermaterial vor dem Aufdampfen bei Temperaturen von 800 bis 1000°C an der Luft erhitzt wird.

5. Palladiumkatalysator nach Anspruch 1, dadurch gekennzeichnet, daß man das Katalysatorgewebe bzw.

die Katalysatorfolie teilweise wellenartig verformt und daraus monolithartige Formkörper herstellt.

6. Verfahren zur Herstellung von Palladiumkatalysatoren, dadurch gekennzeichnet, daß man ein Trägermaterial mit metallischem Palladium bedampft, die aufgedampfte Palladiumschicht mit einem metallischen Inhibitor durch Bedampfen behandelt und eine thermische Behandlung bei Temperaturen von 300 bis 800°C anschließt.

7. Verfahren nach Anspruch 6, dadurch gekennzeichnet, daß man gleichzeitig oder nacheinander die aufzubringenden Metalle Palladium und Wismut im Vakuum bei $10^{-3}$ bis $10^{-10}$ bar verdampft und auf das Trägermaterial niederschlägt.

8. Verwendung der Palladiumkatalysatoren nach Anspruch 1 zur selektiven Hydrierung einer Dreifachbindung zur olefinischen Doppelbindung.

9. Verwendung der Palladiumkatalysatoren nach Anspruch 1 zur Herstellung von Hydro-Linalool durch selektive Hydrierung von Hydro-Dehydrolinalool.

## Revendications

1. Catalyseur au palladium, pouvant être obtenu par métallisation sous vide d'une matière de support avec du palladium métallique, traitement de la couche de palladium déposée par un inhibiteur métallique par métallisation sous vide, puis traitement thermique à des températures de 300 à 800°C.

2. Catalyseur au palladium selon la revendication 1, caractérisé en ce que la matière de support est un acier spécial contenant du fer, du chrome et/ou de l'aluminium.

3. Catalyseur au palladium selon la revendication 1, caractérisé en ce que la matière de support est en forme de treillis, de tissu ou de feuille.

4. Catalyseur au palladium selon la revendication 1, caractérisé en ce qu'avant la métallisation sous vide, la matière de support est chauffée à l'air à une température de 800 à 1000°C.

5. Catalyseur au palladium selon la revendication 1, caractérisé en ce qu'on donne au tissu de catalyseur ou à la feuille de catalyseur une forme partiellement ondulée et on fabrique des corps façonnés monolithiques à partir de ce tissu ou de cette feuille.

6. Procédé de préparation de catalyseurs au palladium, caractérisé en ce qu'une matière de support est métallisée sous vide avec du palladium métallique, la couche de palladium déposée est traitée par un inhibiteur métallique par métallisation sous vide, et traitement thermique à des températures de 300 à 800°C fait suite.

7. Procédé selon la revendication 6, caractérisé en ce qu'on vaporise et on dépose simultanément ou successivement sur la Matière de support les étaux palladium et bismuth à appliquer, sous un vide de $10^{-3}$ à $10^{-10}$ bar.

8. Utilisation des catalyseurs au palladium selon la revendication 1 pour l'hydrogénation sélective d'une triple liaison en double liaison oléfinique.

9. Utilisation des catalyseurs au palladium selon la revendication 1 pour la préparation d'hydrolinalol par hydrogénation sélective d'hydro-déshydrolinalol.

## Claims

1. A palladium catalyst which can be obtained by vapor-depositing metallic palladium on a carrier, treating the palladium coating with a metallic inhibitor by vapor deposition, and heating the product at from 300 to 800°C.

2. A palladium catalyst as claimed in claim 1, wherein the carrier is iron-, chromium- and/or aluminum- containing stainless steel.

3. A palladium catalyst as claimed in claim 1, wherein the carrier is in the form of a mesh, fabric or foil.

4. A palladium catalyst as claimed in claim 1, wherein the carrier is heated in air at from 800 to 1000°C before the vapor deposition.

5. A palladium catalyst as claimed in claim 1, wherein the catalyst fabric or foil is partly waved, and a monolith is produced therefrom.

6. A process for the preparation of a palladium catalyst, which comprises vapor depositing metallic palladium on a carrier, treating the palladium coating with a metallic inhibitor by vapor deposition, and thermally treating the product at from 300 to 800°C.

7. A process as claimed in claim 6, wherein the metals palladium and bismuth are simultaneously or consecutively evaporated at a reduced pressure of from $10^{-3}$ to $10^{-10}$ bar, and precipitated onto the carrier.

8. Use of a palladium catalyst as claimed in claim 1 for selectively hydrogenating a triple bond to an olefinic double bond.

9. Use of a palladium catalyst as claimed in claim 1 for preparing hydrolinalool by selective hydrogenation of hydrodehydrolinalool.